# EUROPEAN PATENT SPECIFICATION

(11) **EP 3 409 261 B1**
(45) Date of publication and mention of the grant of the patent: **26.07.2023**
(21) Application number: 17743881.9
(22) Date of filing: 05.01.2017
(51) Int. Cl.: A61K 6/833, A61K 6/853

(54) **DENTAL INVESTMENT MATERIAL**
DENTALES EINBETTMATERIAL
MATÉRIAU DE REVÊTEMENT DENTAIRE

(30) Priority: 26.01.2016 JP 2016012801
(43) Date of publication of application: 05.12.2018
(73) Proprietor: GC Corporation, Sunto-gun, Shizuoka 410-1307 (JP)
(72) Inventor: MORI, Daizaburo, Tokyo 174-8585 (JP); YOSHINAGA, Masatoshi, Tokyo 174-8585 (JP); MASHIO, Go, Tokyo 174-8585 (JP); FUJIMOTO, Tatsuya, Tokyo 174-8585 (JP); YOKOHARA, Hayato, Tokyo 174-8585 (JP); HOSHINO, Tomohiro, Tokyo 174-8585 (JP); MIYAKE, Takahiro, Tokyo 174-8585 (JP)
(74) Representative: Müller-Boré & Partner Patentanwälte PartG mbB
(86) International application number: PCT/JP2017/000092
(87) International publication number: WO 2017/130647

(56) References cited:
- JP-A- S5 049 116
- JP-A- S6 219 236
- JP-A- S6 287 427
- JP-A- H08 104 535
- JP-A- S61 209 921
- JP-A- S62 288 120
- JP-A- S62 288 121
- US-A1- 2014 106 956
- US-A1- 2015 258 602
- R. V. CURTIS: "Stress-strain and thermal expansion characteristics of a phosphate-bonded investment mould material for dental super plastic forming", JOURNAL OF DENTISTRY, vol. 26, no. 3, 1998, pages 251-258, XP002793522,

## Description

### TECHNICAL FIELD

The present invention relates to a dental investment.

### BACKGROUND ART

When a part of a tooth is removed or lost by a dental treatment or the like, as it is impossible for the tooth to naturally regrow the removed portion or the lost portion, a dental prosthesis is provided at the removed portion or the lost portion. As a material of a dental prosthesis, conventionally, metal is used. However, from a viewpoint of avoiding metal material due to metal allergy, or from an aesthetic viewpoint, a demand for a dental prosthesis made of ceramic material is increasing in recent years.

Among ceramics materials, lithium silicate-based glass ceramics having high strength and an aesthetic property are widely used as materials for dental prostheses in recent years. Methods of molding lithium silicate-based glass ceramics into dental prosthesis are roughly divided into two categories. One category is a mechanical processing method in which a cutting process and the like are performed on block-shaped glass ceramics by a processing machine, and the other category is a press molding method in which a molding process is performed by applying high pressure to glass ceramics in a mold.

In the press molding method, a wax pattern having a shape of a dental prosthesis is produced, and the wax pattern is embedded into the dental investment. After the dental investment hardens, the wax pattern is burned. Thereby, a mold having a cavity of the shape of the dental prosthesis is obtained.

Next, using a heating/pressing molding machine for dental ceramics, ingot state glass ceramics is heated to a temperature lower by about 30°C to 50°C than the melting point of the glass ceramics to be in a softened state. Then, the glass ceramics is pushed into the cavity of the mold to perform a molding process with high pressure. These processes are required to be performed when the glass ceramics is in a softened state. This is because at a temperature higher than the melting point of glass ceramics, the glass ceramics is in a molten state, loses its crystallinity, and is in an amorphous glass state. By molding glass ceramics in a softened state, a crystalline state of the glass ceramics can be maintained, whereby a color tone suitable for a dental prosthesis can be obtained. Also, in order to obtain a dental prosthesis having high strength, high pressing pressure is required.

For example, Patent Document 1 discloses a method of producing a sintered ceramic dental prosthesis, in which in a muffle including a pressing channel and at least one mold cavity that is connected to the pressing channel via at least one connection channel, while heating a raw material for pressing inserted in the pressing channel and applying a pressing pressure to the raw material for pressing, a material substance of the raw material for pressing is filled into the mold cavity.

Patent Documents 2 and 3 disclose further dental investments comprising boron nitride.

### [Prior Art Document]

### [Patent Document]

[Patent Document 1] Japanese Laid-open Patent Publication No. 2009-112818
[Patent Document 2] US 2014/106956 A1
[Patent Document 3] US 2015/258602 A1

### DISCLOSURE OF THE INVENTION

### PROBLEM TO BE SOLVED BY THE INVENTION

As described above, in a case where a dental prosthesis made of glass ceramics is produced by a press molding method, heated and softened glass ceramics is pushed into a mold made of a dental investment under a high pressure, and very high pressure is applied between the dental investment and the glass ceramics. Thus, in a mold made of a conventional dental investment, there is a problem in which a reaction occurs between the dental investment and the glass ceramics, the inner surface of the mold is rough, and a surface property of the obtained dental prosthesis is rough.

An object of the present invention is to provide a dental investment that can withstand high pressure for when producing a dental prosthesis made of glass ceramics by a press molding method and by which a dental prosthesis having a favorable surface property can be obtained.

### MEANS FOR SOLVING THE PROBLEM

The inventors of the present invention have earnestly conducted an investigation in order to solve the above described problem. As a result, the inventors of the present invention have found that a phosphate-bonded investment including boron nitride at a predetermined amount solves the above described problem and accomplished the present invention.

A dental investment according to the present invention is a dental phosphate-bonded investment including boron nitride as defined in the claims.

### EFFECTS OF THE INVENTION

By using a dental investment according to the present invention, it is possible to withstand high pressure for when producing a dental prosthesis made of glass ceramics by a press molding method and to obtain a dental prosthesis having a favorable surface property.

### EMBODIMENT FOR CARRYING OUT THE INVENTION

In the following, an embodiment for carrying out the present invention will be described. The present invention is not limited to the embodiment described below or the like, and various modifications and substitutions may be made for the embodiment described below without departing from the scope recited in claims.

A dental investment according to the present embodiment is a phosphate-bonded investment. The phosphate-bonded investment includes silica (silicon dioxide), ammonium phosphate, and magnesium oxide as main components. Because a dental investment according to the present embodiment is a phosphate-bonded investment, a mold is not destroyed or deformed even under high temperature and high pressure conditions at the time of press molding, and a dental prosthesis having a desired shape can be obtained.

In the phosphate-bonded investment, the contained amount of silica is approximately in a range of from 55% to 85% by weight, the contained amount of ammonium phosphate is approximately in a range of from 10% to 30% by weight, and the contained amount of magnesium oxide is approximately in a range of from 5% to 15% by weight.

The dental investment according to the present embodiment includes boron nitride at 0.1% to 5% by weight. Because the dental investment according to the present embodiment includes boron nitride at 0.1% to 5% by weight, even under high temperature and high pressure conditions at the time of press molding, it is possible to suppress a reaction between the dental investment and glass ceramics, and to suppress roughness of an inner surface of a mold. The contained amount of boron nitride in the dental investment is preferably in a range of from 0.1% to 5%. If the contained amount of boron nitride is less than 0.1% by weight, the effect of suppressing roughness of the inner surface of a mold is low. If the contained amount of boron nitride exceeds 5% by weight, strength as a mold is decreased and it is impossible to withstand high pressure at the time of press molding, and the mold is easily destroyed. The contained amount of boron nitride in the dental investment is more preferably in a range of from 0.5% to 3%.

Also, in a case where the particle size of boron nitride is large, the inner surface of a mold is likely to become rough. Conversely, fine boron nitride whose average particle diameter is less than or equal to 0.1 µm is expensive, which increases the cost of a dental investment. Therefore, the average particle diameter of boron nitride is in a range of from 1 µm to 20 um. Note that in the present specification, the "average particle diameter" means a particle diameter at an integrated value 50% in a particle size distribution of particles obtained by a laser diffraction/scattering method.

In a dental investment according to the present invention, a pigment, an expansion adjusting agent, and the like may be mixed as needed.

A dental investment according to the present invention exerts a high effect on the press molding of ceramic material, particularly glass ceramics. However, the use of a dental investment according to the present invention is not limited to this. For example, a dental investment according to the present invention is also useful for the casting of a conventional metal material.

In the following, dental investments according to the present invention will be described with reference to specific examples. The present invention is not limited to these examples.

### Examples

### <Preparation of dental investments>

At compositions indicated in Table 1, dental investments of examples 1 to 5 and comparative examples 1 to 6 were prepared. Note that the unit of each component is % by weight.

### <Press molding test>

Next, a press molding test was performed on each prepared dental investment according to the following procedure.
1. 20% by weight of a colloidal silica aqueous solution was mixed with each of the dental investments of examples 1 to 5 and comparative examples 1 to 6 at a proportion in which the colloidal silica aqueous solution was 25 mL with respect to 100 g of the dental investment. Thereby, each dental investment slurry was prepared.
2. An acrylic disk having a diameter of 20 mm was embedded with each dental investment slurry.
3. After each dental investment hardened, each dental investment slurry was placed in an electric furnace at 850°C for 60 minutes to burn up the acrylic disc to obtain a mold.
4. Using obtained each mold, press molding of a lithium disilicate ingot (e.max HT A2; manufactured by Ivoclar Vivadent Corporation) was conducted under the following conditions.
   Start temperature: 700°C
   Heating rate: 60°C/min
   Final temperature: 930° C, 15 minutes
   Pressing pressure: 220 N
   Pressing time: 3 minutes
   Heating/press molding machine: GC PANAMAT PRESS (manufactured by GC Corporation)

Composition of lithium disilicate ingot (e.max HT A2):
SiO₂: 57% to 80% by weight
Li₂O: 11% to 19% by weight
K₂O: 0% to 13% by weight
P₂O₅: 0% to 11% by weight
ZrO₂: 0% to 8% by weight
ZnO: 0% to 8% by weight

Other oxides and ceramic pigment: 0% to 10% by weight 5. Each glass ceramic disc cast by the press molding was recovered from the mold, and sandblasting was performed on the surface of each glass ceramic disc (casting) using glass beads. Thereafter, the surface roughness Ra was measured using a three-dimensional shape measurement device (VR-3100; manufactured by Keyence Corporation), and the surface property of each casting was evaluated with the following index. The evaluation results are indicated in Table 1.
A: Surface roughness Ra is less than 8 µm
B: Surface roughness Ra is from 8 µm to 15 µm
C: Surface roughness Ra exceeds 15 µm

Also, the presence or absence of destruction of each mold during the press molding was evaluated using the following index. The evaluation results are indicated in Table 1.
OK: No problem
NG: Destruction of the mold was found

**[Table 1]**

| (UNIT: % BY WEIGHT) | | EXAMPLE 1 | EXAMPLE 2 | EXAMPLE 3 | EXAMPLE 4 | EXAMPLE 5 | COMPARATIVE EXAMPLE 1 | COMPARATIVE EXAMPLE 2 | COMPARATIVE EXAMPLE 3 | COMPARATIVE EXAMPLE 4 | COMPARATIVE EXAMPLE 5 | COMPARATIVE EXAMPLE 6 |
|---|---|---|---|---|---|---|---|---|---|---|---|---|
| SILICON DIOXIDE (SILICA) | | 82 | 69 | 77 | 76 | 65 | 69 | 69 | 69 | 69 | 60 | 40 |
| AMMONIUM PHOSPHATE | | 12 | 20 | 14 | 16 | 20 | 20 | 20 | 20 | 21 | 20 | 20 |
| MAGNESIUM OXIDE | | 5.5 | 10 | 7 | 7 | 10 | 10 | 10 | 10 | 10 | 10 | 10 |
| BORON NITRIDE | (AVERAGE PARTICLE DIAMETER 12 *µ*m) | 0.5 | 1 | 2 | | | | | | | 10 | 30 |
| | (AVERAGE PARTICLE DIAMETER 2 *µ*m) | | | | 1 | 5 | | | | | | |
| MOLYBDENUM DISULFIDE | | | | | | | 1 | | | | | |
| GRAPHITE | | | | | | | | 1 | | | | |
| POLYTETRAFLUOROETHYLENE | | | | | | | | | 1 | | | |
| EVALUATION RESULT | SURFACE PROPERTY OF CASTING | B | A | A | A | B | C | C | C | C | C | C |
| | CRACK OF MOLD | OK | OK | OK | OK | OK | OK | OK | OK | OK | NG | NG |

As can been seen from Table 1, in a case where press molding of glass ceramics is performed using a dental investment according to the present invention, it is possible to obtain a casting having a favorable surface property.

In contrast, in a case where an investment does not include boron nitride but includes, as a release agent, molybdenum disulfide (comparative example 1), graphite (comparative example 2), or polytetrafluoroethylene (comparative example 3), a casting having a favorable surface property could not be obtained. Similarly, in a case where an investment includes none of boron nitride and a release agent, a casting having a favorable surface property could not be obtained (comparative example 4). Further, when the content of boron nitride in an investment is excessive, the mold made of the investment was destroyed by high pressure at the time of press molding due to insufficient strength, and a casting having a desired shape could not be obtained (comparative examples 5 and 6).

## Claims

1. A dental investment that is a dental phosphate-bonded investment including boron nitride at 0.1% to 5% by weight,
wherein an average particle diameter of the boron nitride is in a range of from 1 *µ*m to 20 *µ*m at an integrated value 50% in a particle size distribution obtained by a laser diffraction/scattering method.

2. The dental investment according to claim 1, further comprising:
silicon dioxide,
ammonium phosphate, and
magnesium oxide,
wherein a content of the silicon dioxide is in a range of from 55% to 85% by weight,
wherein a content of the ammonium phosphate is in a range of from 10% to 30% by weight, and
wherein a content of the magnesium oxide is in a range of from 5% to 15% by weight.

3. The dental investment according to claim 1, wherein a content of the boron nitride is in a range of from 0.5% to 3% by weight.

## Patentansprüche

1. Dentaleinbettmaterial, das ein Phosphat-gebundenes Dentaleinbettmaterial ist, das 0,1 Gew.-% bis 5 Gew.-% Bornitrid umfasst,
wobei der durchschnittliche Teilchendurchmesser des Bornitrids in einem Bereich von 1 µm bis 20 µm bei einem 50 %-Integrationswert in einer Teilchengrößenverteilung liegt, die durch ein Laserbeugungs/Streuungsverfahren erhalten wird.

2. Dentaleinbettmaterial nach Anspruch 1, ferner umfassend:
Siliziumdioxid,
Ammoniumphosphat und
Magnesiumoxid,
wobei der Gehalt des Siliziumdioxids in einem Bereich von 55 Gew.-% bis 85 Gew.-% liegt,
wobei der Gehalt des Ammoniumphosphats in einem Bereich von 10 Gew.-% bis 30 Gew.-% liegt, und
wobei der Gehalt des Magnesiumoxids in einem Bereich von 5 Gew.-% bis 15 Gew.-% liegt.

3. Dentaleinbettmaterial nach Anspruch 1, wobei der Gehalt des Bornitrids in einem Bereich von 0,5 Gew.-% bis 3 Gew.-% liegt.

## Revendications

1. Revêtement dentaire qui est un revêtement dentaire à liant phosphate comportant 0,1 % à 5 % en poids de nitrure de bore,
dans lequel un diamètre de particule moyen du nitrure de bore se situe dans une plage de 1 µm à 20 µm à une valeur intégrée de 50 % d'une distribution de taille de particule obtenue par une méthode de diffraction/diffusion laser.

2. Revêtement dentaire selon la revendication 1, comprenant en outre :
du dioxyde de silicium
du phosphate d'ammonium, et
de l'oxyde de magnésium,
dans lequel une teneur en dioxyde de silicium se situe dans une plage de 55 % à 85 % en poids,
dans lequel une teneur en phosphate d'ammonium se situe dans une plage de 10 % à 30 % en poids, et
dans lequel une teneur en oxyde de magnésium se situe dans une plage de 5 % à 15 % en poids.

3. Revêtement dentaire selon la revendication 1, dans lequel une teneur en nitrure de bore se situe dans une plage de 0,5 % à 3 % en poids.
